## Europäisches Patentamt

## European Patent Office

## Office européen des brevets

(19)

(11) Veröffentlichungsnummer : **0 026 280**
**B1**

(12) # EUROPÄISCHE PATENTSCHRIFT

(45) Veröffentlichungstag der Patentschrift :
24.11.82

(21) Anmeldenummer : 80104182.3

(22) Anmeldetag : 17.07.80

(51) Int. Cl.³ : **C 07 C 53/12, C 07 C 51/54**

(54) **Verfahren zur Herstellung von Essigsäureanhydrid.**

(30) Priorität : 02.10.79 DE 2939839

(43) Veröffentlichungstag der Anmeldung :
08.04.81 (Patentblatt 81/14)

(45) Bekanntmachung des Hinweises auf die Patenterteilung : 24.11.82 Patentblatt 82/47

(84) Benannte Vertragsstaaten :
AT BE CH DE FR GB IT LI NL

(56) Entgegenhaltungen :
EP A 0 008 396
DE A 2 450 965
DE A 2 658 216

(73) Patentinhaber : HOECHST AKTIENGESELLSCHAFT
Postfach 80 03 20
D-6230 Frankfurt/Main 80 (DE)

(72) Erfinder : Kübbeler, Hans-Klaus, Dr.
Bünnagelring 31
D-5357 Swisttal 8 (DE)
Erfinder : Erpenbach, Heinz, Dr.
Oberbuschweg 22
D-5000 Köln 50 (DE)
Erfinder : Gehrmann, Klaus, Dr.
Geschwister-Scholl-Strasse 32
D-5042 Erftstadt (DE)
Erfinder : Schmitz, Klaus
Rodderweg 46
D-5042 Erftstadt (DE)

EP 0 026 280 B1

## Verfahren zur Herstellung von Essigsäureanhydrid

Die Erfindung betrifft ein Verfahren zur Herstellung von Essigsäureanhydrid durch Umsetzung von Methylacetat und/oder Dimethylether mit Kohlenmonoxid unter praktisch wasserfreien Bedingungen bei Temperaturen von 350 bis 575 K und Drücken von 1 bis 300 bar in Gegenwart eines Katalysatorsystems, welches Edelmetalle der Gruppe VIII des Periodensystems der Elemente oder deren Verbindungen, Jod und/oder dessen Verbindungen, eine aliphatische Carbonsäure mit 1-8 Kohlenstoffatomen sowie eine heterocyclische aromatische Verbindung, in der mindestens ein Heteroatom ein quaternäres Stickstoffatom ist, und deren Schmelz- oder Mischschmelzpunkt unterhalb 413 K, dem Siedepunkt von Essigsäureanhydrid, liegt, oder eine quaternäre Organophosphorverbindung enthält, welches dadurch gekennzeichnet ist, daß das Katalysatorsystem eine im Reaktionsgemisch lösliche Zirkonverbindung enthält.

Die DE-A1-26 58 216 beschreibt bereits ein vergleichbares Verfahren zur Herstellung von Monocarbonsäureanhydriden, jedoch mit dem Unterschied, daß anstelle des Edelmetalls Nickel und Chrom, und außerdem keine Zirkonverbindung eingesetzt werden. Die DE-A1-26 58 216 verwendet weiterhin eine Organostickstoff- oder Organophosphorverbindung mit dreiwertigem Stickstoff bzw. Phosphor als Promotor.

Die DE-A1-24 50 965 betrifft ein Verfahren zur Herstellung von Essigsäureanhydrid aus Essigsäuremethylester und Kohlenmonoxid, wobei man die Reaktionspartner bei Drücken von 1-500 bar und Temperaturen von 50 bis 250 °C über Katalysatoren leitet, die Edelmetalle der 8. Nebengruppe des Periodensystems oder deren Verbindungen sowie Jod und/oder Jodverbindungen enthalten. Als Jodverbindungen werden auch quaternäre Ammonium- oder Phosphoniumverbindungen wie Tetramethylammoniumjodid und Tetraethylphosphoniumjodid genannt. Zirkonverbindungen sind im Katalysatorsystem jedoch nicht enthalten.

Die DE-A1-26 10 036 beschreibt ebenfalls ein vergleichbares Verfahren zur Herstellung von Monocarbonsäureanhydriden, jedoch mit dem Unterschied, daß neben dem Edelmetall der Gruppe VIII des Periodensystems und einem Jodid ein Mehrfachpromotor eingesetzt wird, der ein Metall, vorzugsweise Chrom, sowie eine Organostickstoff- oder Organophosphorverbindung mit dreibindigem Stickstoff bzw. Phosphor enthält.

Beim Verfahren der DE-A1-26 10 036 wirkt es sehr störend, daß die Metallverbindungen und Folgeprodukte des Mehrfachpromotors in siedendem Acetanhydrid weitgehend unlöslich sind, so daß die beim kontinuierlichen Verfahren erforderliche Kreislaufführung des Katalysatorsystems seh erschwert, wenn nicht gar unmöglich gemacht wird. Ebenso beeinträchtigen diese unlöslichen Verbindungen die Trennung des Acetanhydrids vom Katalysatorsystem in unzulässigem Maße. So tritt beispielsweise bei der destillativen Aufarbeitung des Reaktionsgemisches eine Belegung des Verdampfers mit grünen Chromsalzen auf, wodurch der Wärmeübergang empfindlich gestört ist. Die infolgedessen notwendige Steigerung der Verdampfertemperatur führt zu einer erheblichen Schädigung des Katalysatorsystems. Als Folge davon muß eine langwierige, verlustreiche Zwischenbehandlung des teuren, edelmetallhaltigen Katalysators vorgenommen werden, wobei die auftretenden Katalysatorverluste eine schnelle Aktivitätsminderung im gesamten System nach sich ziehen. Diese Umstände verhinderten bischer die Durchführung des Verfahrens im technischen Maßstab.

Die ältere EP-A1-8396 (DE-A1-28 36 084) beschreibt bereits Lösungen zur Beseitigung der oben geschilderten Nachteile. Anstelle des Systems aus Chromverbindungen und Organostickstoff- oder Organophosphorverbindungen mit dreibindigem Stickstoff bzw. Phosphor wird hier eine heterocyclische aromatische Verbindung mit quaternärem Stickstoff in Kombination mit einer aliphatischen Carbonsäure eingesetzt. Derartige Addukte mit quaternärem Stickstoff sind unter Reaktionsbedingungen schmelzflüssig und stören die Kreislaufführung des Katalysatorsystems in keiner Weise. Ebensowenig wird durch diesen Austausch die Selektivität des Katalysatorsystems herabgesetzt, vielmehr wird durch die getroffenen Maßnahmen die Aktivität des Katalysatorsystems beträchtlich erhöht. Die hierbei eingesetzten heterocyclischen aromatischen Verbindungen mit mindestens einem quaternären Stickstoffatom als Heteroatom bilden einzeln oder im Gemisch sowohl unter Reaktionsbedingungen als auch unter den Bedingungen der Aufarbeitung der Reaktionsprodukte der Carbonylierung von Methylacetat und Dimethylether Schmelzen, die sich als geeignete Lösemittel für die Edelmetallkomplexe erweisen und darüber hinaus mit Essigsäureanhydrid gut mischbar sind.

Die eingangs geschilderte Erfindung bewirkt nun eigenartigerweise eine bedeutende Steigerung der Katalysatoraktivität, ausgedrückt in Gramm erhaltenen Essigsäureanhydrids je Gramm elementares Edelmetall und Stunde.

Diese Beobachtung ist um so überraschender, als die Löslichkeit bestimmter Zirkonverbindungen im Reaktionssystem nicht vorauszusehen war und aus der Literatur Beispiele für eine derartige Sonderstellung von Zirkonverbindungen bei der Carbonylierung von Estern und/oder Ethern nicht bekannt sind.

Im Rahmen der Erfindung verwendbare Zirkonverbindungen sind z.B. Zirkonhalogenid, Zirkonylhalogenid, Zirkonacetat, Zirkonylacetat, Organozirkonverbindungen wie Bis (cyclopentadienyl) zirkondihalogenid. Als Halogenide bewähren sich die Chloride, Bromide und Jodide.

Die Erfindung ist bevorzugt und wahlweise dadurch gekennzeichnet, daß man

a) Organophosphoverbindungen einsetzt, deren Schmelz- oder Mischschmelzpunkte unterhalb 413 K, dem Siedepunkt von Essigsäureanhydrid, liegen ;

b) die heterocyclischen Verbindungen oder Organophosphorverbindungen in Form ihrer Addukte mit Essigsäure oder Methyljodid einsetzt ;

c) das Katalysatorsystem Edelmetall (-verbindung)/Zirkonverbindung/Jod (-verbindung)/-Carbonsäure/heterocyclische Verbindung oder Organophosphorverbindung im Atom- bzw. Molverhältnis 1 : (0,1-10) (1-1 400) : (10-2 000) : (1-1 200) einsetzt ;

d) Kohlenmonoxid/Wasserstoffgemische mit bis zu 20 Volum% Wasserstoff einsetzt.

Als heterocyclische aromatische Verbindungen und als quaternäre Organophosphorverbindungen, die sowohl unter Reaktionsbedingungen als auch unter den Bedingungen der Aufbeitung der Reaktionsprodukte der Carbonylierung von Methylacetat bzw. Dimethylether Schmelzen bilden, die sich als geeignete Lösemittel für die Edelmetallkomplexe und die Zirkonverbindungen· erweisen und darüber hinaus mit Essigsäureanhydrid gut mischbar sind, kommen erfindungsgemäß beispielsweise infrage :

1. N-Methylpyridiniumjodid, N,N-Dimethylimidazoliumjodid, N-Methyl-3-picoliniumjodid, N-Methyl-2,4-lutidiniumjodid, N-Methyl-3,4-lutidiniumjodid, N-Methyl-chinoliniumjodid ;

2. Tributyl-methyl-phosphoniumjodid, Trioctyl-methyl-phosphoniumjodid, Trilauryl-methyl-phosphoniumjodid, Triphenyl-methyl-phosphoniumjodid ;

3. Pyridiniumacetat, N-Methylimidazoliumacetat, 3-Picoliniumacetat, 2,4-Lutidiniumacetat, 3,4-Lutidiniumacetat.

Ggf. kann es vorteilhaft sein, in Gegenwart eines Katalysatorsystems zu arbeiten, welches mehrere heterocyclische aromatische Verbindungen oder mehrere quaternäre Organophosphorverbindungen enthält.

Die Promotoreneigenschaft dieser Addukte wird durch die Anwesenheit einer aliphatischen Carbonsäure mit 1-8 Kohlenstoffatomen erheblich verstärkt.

Bevorzugt wird das Verfahren der Erfindung bei Temperaturen von 400-475 K und Drücken von 20-150 bar durchgeführt. Je Mol Methylacetat und/oder Dimethylether setzt man vorzugsweise 0,000 1 bis 0,01 Mol des Edelmetalls der Gruppe VIII des Periodensystems der Elemente oder seiner Verbindungen ein. Vorzugsweise setzt man das Katalysatorsystem Edelmetall (-verbindung)/Zirkonverbindung/Jod (-verbindung)/Carbonsäure/heterocyclische Verbindung oder Organophosphorverbindung im Atom- bzw. Molverhältnis 1 : (0,5-8) : (10-300) : (25-600) : (10-300) ein. Der Einsatz von Essigsäure als Carbonsäure wird bevorzugt.

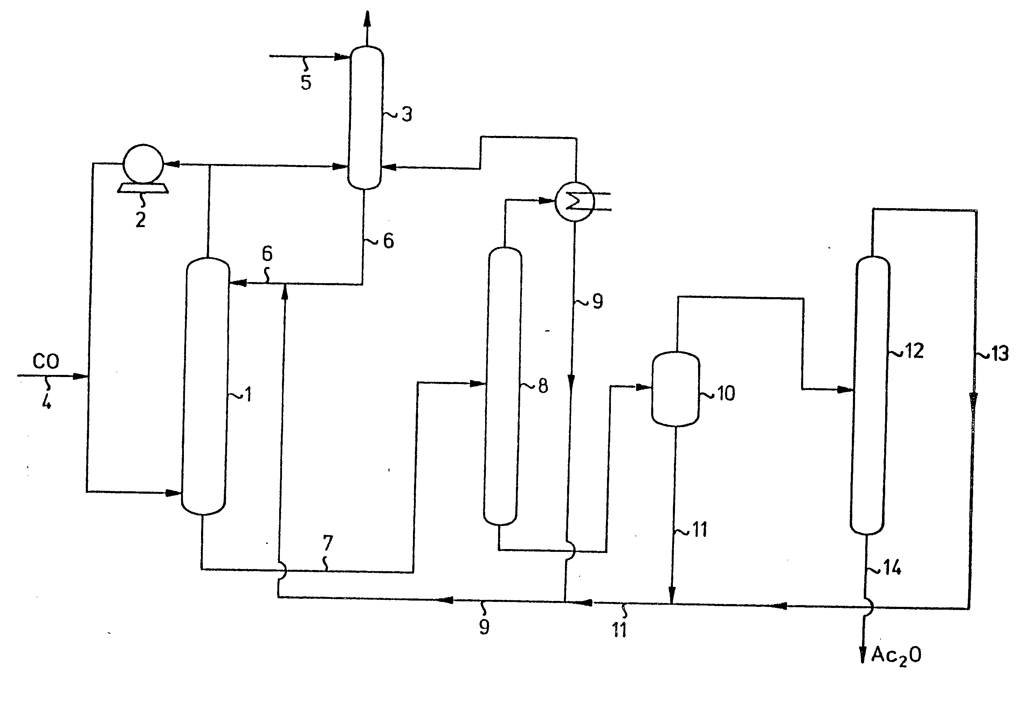

Ein mögliches Fließschema zur Durchführung des erfindungsgemäßen Verfahrens ist in der Zeichnung schematisch dargestellt und im folgenden beschrieben :

In einem Druckreaktor 1 aus Hastelloy C werden Methylacetat und/oder Dimethylether und Kohlenmonoxid oder ein Gemisch aus CO und $H_2$ mit bis zu 20 Vol% $H_2$ in Gegenwart eines Katalysatorsystems aus Edelmetallen der Gruppe VIII des Periodensystems der Elemente oder deren Verbindungen und Jod und/oder dessen Verbindungen, vorzugsweise Methyljodid, unter Zusatz von im Reaktionsgemisch löslichen Zirkonverbindungen sowie in Gegenwart einer Carbonsäure, vorzugsweise Essigsäure, und mindestens einer heterocyclischen aromatischen Verbindung, in der mindestens ein Heteroatom ein quaternäres Stickstoffatom ist, oder einer quaternären Organophosphorverbindung unter einem Druck von vorzugsweise 20 bis 150 bar und einer Temperatur von vorzugsweise 400 bis 475 K zu Acetanhydrid umgesetzt.

Nichtumgesetztes Kohlenmonoxid und ggf. Wasserstoff werden über eine Gasumwälzpumpe 2 im Kreislauf geführt, während ein geringer Teilstrom das System als Abgas über eine Wäsche 3 verläßt. Frisches, ggf. wasserstoffhaltiges Kohlenmonoxid wird dem Umsatz entsprechend über Leitung 4 dem Kreislaufgas zudosiert. Die dem Umsatz entsprechende Menge frischen Methylacetats und/oder Dimethylethers wird über Leitung am Kopf der Wäsche 3 aufgegeben und über Leitung 6 dem Reaktor 1 zugeleitet. Das Reaktionsgemisch strömt über Leitung 7 aus dem Reaktor 1 ab. In der Destillationskolonne 8 erfolgt die Abtrennung der Leichtsieder (Methylacetat bzw. Dimethylether, Methyljodid), die über Leitung 9 und 6 dem Reaktor 1 wieder zugeführt werden. Der Sumpf der Leichtsiederkolonne 8 wird im Verdampfer 10 in Destillat und Katalysator zerlegt. Letzterer gelangt über die Leitungen 11, 9 und 6 zum erneuten Einsatz in den Reaktor 1. Das Destillat des Verdampfers 10 wird in der Destillationskolonne 12 in Essigsäure, die über die Leitungen 13, 11, 9 und 6 in den Reaktor 1 zurückfließt, und in das hergestellte Acetanhydrid, das über Leitung 14 abgenommen wird, getrennt.

Beispiel 1

250 g Methylacetat, 1,6 g $RhCl_3 \cdot 3H_2O$, 5 g $ZrCl_4$, 60 g $CH_3J$, 70 g Essigsäure und 70 g N,N-Dimethylimidazoliumjodid werden in einem Hastelloy-Autoklaven mit CO bei 50 bar und 455 K umgesetzt. Nach einer Reaktionszeit von 15 min werden im Reaktionsgemisch 281 g Essigsäureanhydrid gefunden, entsprechend 1 797 g $Ac_2O$/g Rh · h.

Beispiel 1a

(Vergleichsbeispiel = Beispiel 5 der EP-A1-83 96)

250 g Methylacetat, 1,6 g $RhCl_3 \cdot 3H_2O$, 60 g $CH_3J$, 70 g Essigsäure und 70 g N,N-Dimethylimidazoliumjodid werden in einem Hastelloy-Autoklaven mit CO bei 50 bar und 450 K umgesetzt.

Nach einer Reaktionszeit von 31 min werden im Reaktionsgemisch 283 g Essigsäureanhydrid gefunden, entsprechend 876 g $Ac_2O$/g Rh · h.

**Beispiel 2**

200 g Dimethylether, 1,8 g $RhCl_3 · 3H_2O$, 5 g $ZrCl_4$, 70 g $CH_3J$, 100 g Essigsäure und 80 g N-Methyl-3-picoliniumjodid werden bei 455 K und 60 bar mit CO im Hastelloy-Autoklaven umgesetzt. Nach einer Reaktionszeit von 12 min werden im Reaktionsgemisch 312 g Essigsäureanhydrid gefunden, entsprechend 2 217 g $Ac_2O$/g Rh · h.

Der Dimethylethergehalt im methylacetathaltigen Reaktionsprodukt liegt unter 0,1 Masse-%.

**Beispiel 3**

280 g Methylacetat, 1,8 g $RhCl_3 · 3H_2O$, 8 g $ZrOCl_2 · 8H_2O$, 90 g $CH_3J$, 70 g Essigsäure und 70 g N,N-Dimethylimidazoliumjodid werden in einem Autoklaven mit Zirkonauskleidung mit CO bei 50 bar und 450 K umgesetzt. Nach einer Reaktionszeit von 14 min werden 286 g Essigsäureanhydrid gefunden, entsprechend 1 742 g $Ac_2O$/g Rh · h.

**Beispiel 4 (nicht gemäß der Erfindung)**

250 g Methylacetat, 1,6 g $RhCl_3 · 3H_2O$, 3 g $ZrO_2$, 60 g $CH_3J$, 60 g Essigsäure und 80 g N-Methyl-3-picoliniumjodid werden in einem Hastelloy-Autoklaven mit CO bei 60 bar und 450 K umgesetzt. Nach einer Reaktionszeit von 32 min werden 281 g Essigsäureanhydrid erhalten, entsprechend 843 g $Ac_2O$/g Rh · h. Die Katalysatorleistung macht deutlich, daß ein Zusatz von $ZrO_2$ in Anbetracht des Vergleichsbeispiels 1a ohne Einfluß auf die Aktivität des Katalysatorsystems bleibt, da $ZrO_2$ im Reaktionsmedium unlöslich ist.

**Beispiel 5**

280 g Methylacetat, 1,8 g $IrCl_3$, 8 g $ZrOCl_2 · 8H_2O$, 60 g $CH_3J$, 50 g Essigsäure und 100 g N-Methyl-chinoliniumjodid werden mit CO bei 80 bar und 465 K in einem Hastelloy-Autoklaven umgesetzt. Nach 38 min zeigt die Analyse 273 g Essigsäureanhydrid, entsprechend 372 g $Ac_2O$/g Ir · h.

**Beispiel 6**

260 g Methylacetat, 1,6 g $RhCl_3 · 3H_2O$, 8 g $ZrOCl_2 · 8H_2O$, 70 g $CH_3J$, 60 g Essigsäure und 60 g N,N-Dimethylimidazoliumjodid werden bei 445 K und 60 bar in einem Hastelloy-Autoklaven mit einem Gemisch aus CO und $H_2$, das 8 Vol% $H_2$ enthält, umgesetzt. Es werden nach 18 min 281 g Essigsäureanhydrid im Reaktionsgemisch ermittelt, entsprechend einer Leistung von 1 498 g $Ac_2O$/g Rh · h. Der Ethylidendiacetat-Gehalt des Reaktionsgemisches liegt unter 0,1 Masse-%.

**Beispiel 7**

280 g Methylacetat, 2 g $Pd(OAc)_2$, 10 g $ZrOJ_2 · 8H_2O$, 70 g Essigsäure und 20 g N-Methyl-pyridiniumjodid sowie 40 g N-Methyl-3-picoliniumjodid werden in einem Hastelloy-Autoklaven bei 460 K und 50 bar mit CO umgesetzt. Nach 78 min werden 197 g Essigsäureanhydrid, entsprechend 160 g $Ac_2O$/g Pd · h, erhalten.

**Beispiel 8**

260 g Methylacetat, 1,6 g $RhCl_3 · 3H_2O$, 2 g $ZrOCl_2 · 8H_2O$, 70 g $CH_3J$, 70 g Essigsäure und 60 g N,N-Dimethylimidazoliumjodid werden bei 445 K und 60 bar in einem Hastelloy-Autoklaven mit CO umgesetzt. Es werden nach 24 min 278 g Essigsäureanhydrid ermittelt, entsprechend einer Leistung von 1 110 g $Ac_2O$/g Rh · h.

**Beispiel 9**

250 g Methylacetat, 1,6 g $RhCl_3 · 3H_2O$, 7 g Bis(cyclopentadienyl) zirkondichlorid, 70 g $CH_3J$, 60 g Essigsäure und 60 g N,N-Dimethylimidazoliumjodid werden bei 450 K und 60 bar in einem Hastelloy-Autoklaven mit CO umgesetzt. Nach 14 min werden 292 g Essigsäureanhydrid erhalten, entsprechend einer Leistung von 1 999 g $Ac_2O$/g Rh · h.

**Beispiel 10**

250 g Methylacetat, 1,6 g $RhCl_3 · 3H_2O$, 10 g $ZrOCl_2 · 8H_2O$, 150 g $CH_3J$, 75 g Essigsäure und 100 g N-Methylimidazoliumacetat werden bei 445 K und 60 bar in einem zirkonausgekleidetem Autoklaven mit CO umgesetzt. Nach 19 min werden 284 g Essigsäureanhydrid im Reaktionsgemisch nachgewiesen. Das entspricht einer Leistung von 1 433 g $Ac_2O$/g Rh · h.

**Beispiel 11**

250 g Methylacetat, 0,8 g $RhCl_3 · 3H_2O$, 5 g $ZrCl_4$, 90 g $CH_3J$, 90 g Essigsäure, 120 g N,N-Dimethylimidazoliumjodid und 60 g N-Methyl-3-picoliniumjodid werden im Hastelloy-Autoklaven mit CO bei 70 bar und 445 K umgesetzt. Nach einer Reaktionszeit von 24 min werden 283 g Essigsäureanhydrid festgestellt, entsprechend 2 263 g $Ac_2O$/g Rh · h.

**Beispiel 12**

280 g Methylacetat, 2,7 g $Pd(OAc)_2$, 10 g $ZrOCl_2 · 8H_2O$, 60 g $CH_3J$, 60 g Essigsäure und 200 g Trilauryl-methyl-phosphoniumjodid werden bei 450 K und 80 bar in einem Hastelloy-Autoklaven mit einem Gemisch von CO und $H_2$, das 15 Vol% $H_2$ enthält, umgesetzt. Es werden nach 19 min 278 g Essigsäureanhydrid im Reaktionsgemisch ermittelt, entsprechend einer Leistung von 686 g $Ac_2O$/g Pd · h.

**Beispiel 13**

250 g Methylacetat, 2,7 g Pd(OAc)$_2$, 6 g ZrCl$_4$, 70 g CH$_3$J, 70 g Essigsäure und 150 g Tributyl-methyl-phosphoniumjodid werden bei 460 K und 100 bar in einem Hastelloy-Autoklaven mit einem Gemisch von CO und H$_2$, das 10 Vol% H$_2$ enthält, umgesetzt. Es werden nach 15 min 273 g Essigsäureanhydrid im Reaktionsgemisch festgestellt, entsprechend einer Leistung von 853 g Ac$_2$O/g Pd · h.

**Beispiel 14**

280 g Methylacetat, 0,8 g RhCl$_3$ · 3H$_2$O, 8 g ZrOCl$_2$ · 8H$_2$O, 60 g CH$_3$J, 70 g Essigsäure und 150 g Trioctyl-methyl-phosphoniumjodid werden bei 450 K und 60 bar in einem Hastelloy-Autoklaven mit CO umgesetzt. Nach 35 min werden 281 g Essigsäureanhydrid im Reaktionsgemisch ermittelt, entsprechend einer Leistung von 1 540 g Ac$_2$O/g Rh · h.

**Beispiel 15**

280 g Methylacetat, 2,7 g Pd(OAc)$_2$, 8 g ZrCl$_4$, 60 g CH$_3$J, 70 g Essigsäure, 70 g Tributyl-methyl-phosphoniumjodid und 70 g Methyltriphenylphosphoniumjodid werden bei 460 K und 100 bar in einem Hastelloy-Autoklaven mit einem Gemisch von CO und H$_2$, das 9 Vol% H$_2$ enthält, umgesetzt. Nach 17 min werden in Reaktionsgemisch 299 g Essigsäureanhydrid nachgewiesen, was einer Leistung von 824 g Ac$_2$O/g Pd · h entspricht.

**Ansprüche**

1. Verfahren zur Herstellung von Essigsäureanhydrid durch Umsetzung von Methylacetat und/oder Dimethylether mit Kohlenmonoxid unter praktisch wasserfreien Bedingungen bei Temperaturen von 350 bis 575 K und Drücken von 1 bis 300 bar in Gegenwart eines Katalysatorsystems, welches Edelmetalle der Gruppe VIII des Periodensystems der Elemente oder deren Verbindungen, Jod und/oder dessen Verbindungen, eine aliphatische Carbonsäure mit 1-8 Kohlenstoffatomen sowie eine heterocyclische aromatische Verbindung, in der mindestens ein Heteroatom ein quaternäres Stickstoffatom ist, und deren Schmelz- oder Mischschmelzpunkt unterhalb 413 K, dem Siedepunkt von Essigsäureanhydrid, liegt, oder eine quaternäre Organophosphorverbindung enthält, dadurch gekennzeichnet, daß das Katalysatorsystem eine im Reaktionsgemisch lösliche Zirkonverbindung enthält.

2. Verfahren nach Anspruch 1, dadurch gekennzeichnet, daß man Organophosphorverbindungen einsetzt, deren Schmelz- oder Mischschmelzpunkte unterhalb 413 K, dem Siedepunkt von Essigsäureanhydrid, liegen.

3. Verfahren nach einem der vorhergehenden Ansprüche, dadurch gekennzeichnet, daß man die heterocyclischen Verbindungen oder Organophosphorverbindungen in Form ihrer Addukte mit Essigsäure oder Methyljodid einsetzt.

4. Verfahren nach einem der vorhergehenden Ansprüche, dadurch gekennzeichnet, daß man das Katalysatorsystem Edelmetall (-verbindung)/Zirkonverbindung/Jod (-verbindung)/Carbonsäure/heterocyclische Verbindung oder Organophosphorverbindung im Atom- bzw. Molverhältnis 1 : (0,1-10) : (1-1 400) : (10-2 000) : (1-1 200) einsetzt.

5. Verfahren nach einem der vorhergehenden Ansprüche, dadurch gekennzeichnet, daß man Kohlenmonoxid/Wasserstoffgemische mit bis zu 20 Volum% Wasserstoff einsetzt.

**Claims**

1. Process for making acetic anhydride by reacting methyl acetate and/or dimethylether with carbon monoxide under practically anhydrous conditions, at temperatures of 350 to 575 K, under pressures of 1 to 300 bars, and in the presence of a catalyst system containing noble metals belonging to group VIII of the periodic system of the elements, or their compounds, iodine and/or its compounds, an aliphatic carboxylic acid having 1 to 8 carbon atoms, as well as a heterocyclic aromatic compound, in which at least one hetero atom is a quaternary nitrogen atom and which has a melting point or mixed melting point of less than 413 K, the boiling point of acetic anhydride, or a quaternary organophosphorus compound, characterized in that the catalyst system contains a zirconium compound soluble in the reaction mixture.

2. Process as claimed in claim 1, wherein organophosphorus compounds are used, of which the melting points or mixed melting points are lower than 413 K, the boiling of acetic anhydride.

3. Process as claimed in any of the preceding claims, wherein the heterocyclic compounds or organophosphorus compounds are used in the form of their addition products with acetic acid or methyl iodide.

4. Process as claimed in any of the preceding claims, wherein the catalyst system comprised of noble metal (compound)/zirconium compound/iodine (compound)/carboxylic acid/heterocyclic compound or organophosphorus compound is used in an atomic or molar ratio of 1 : (0.1-10) : (1-1 400) : (10-2 000) : (1-1 200).

5. Process as claimed in any of the preceding claims, wherein a carbon monoxide/hydrogen-mixture containing up to 20 volume% of hydrogen is used.

**Revendications**

1. Procédé de préparation de l'anhydride acétique par réaction de l'acétate de méthyle et/ou de l'éther diméthylique avec l'oxyde de carbone, dans des conditions pratiquement anhydres, à des températures de 350 à 575 °K, sous des pressions de 1 à 300 bars, en présence d'un système catalyseur qui contient des métaux nobles du groupe VIII de la Classification Périodique des Eléments ou leurs composés, de l'iode et/ou ses composés, un acide carboxylique aliphatique en $C_1$-$C_8$ et un composé aromatique hétérocyclique dans lequel au moins un hétéroatome est un atome d'azote quaternaire et dont le point de fusion ou le point de fusion en mélange est inférieur à 413 °K, le point d'ébullition de l'anhydride acétique, ou un composé organophosphoré quaternaire, caractérisé en ce que le système catalyseur contient un composé de zirconium soluble dans le mélange de réaction.

2. Procédé selon la revendication 1, caractérisé en ce que l'on utilise des composés organophosphorés dont le point de fusion ou le point de fusion en mélange est inférieur à 413 °K, le point d'ébullition de l'anhydride acétique.

3. Procédé selon l'une des revendications qui précèdent, caractérisé en ce que l'on utilise les composés hétérocycliques ou les composés organophosphorés à l'état de leurs adducts avec l'acide acétique ou l'iodure de méthyle.

4. Procédé selon l'une des revendications qui précèdent, caractérisé en ce que l'on utilise le système catalyseur métal noble (composé de)/composé du zirconium/iode (composé de)/acide carboxylique/composé hétérocyclique ou composé organophosphoré à des proportions relatives atomiques ou molaires respectivement de 1 : (0,1 à 10) : (1 à 1 400) : (10 à 2 000) : (1 à 1 200).

5. Procédé selon l'une des revendications qui précèdent, caractérisé en ce que l'on utilise des mélanges oxyde de carbone/hydrogène contenant jusqu'à 20 % en volume d'hydrogène.

CO

$Ac_2O$

0 026 280